# EUROPEAN PATENT APPLICATION

(11) **EP 1 421 942 A1**
(43) Date of publication of application: **26.05.2004**
(21) Application number: 03256759.6
(22) Date of filing: 27.10.2003
(51) Int. Cl.: A61K 31/704, A61K 31/235, A61K 31/19, A61P 31/04, A61P 31/18, A61P 35/00

(54) **Use of glycyrrhizin and its derivatives as RANTES inducers**

(30) Priority: 29.10.2002 US 422339 P
(71) Applicant: Minophagen Pharmaceutical Co., Ltd., Tokyo (JP)
(72) Inventor: Kobayashi, Makiko, Galveston Texas 77550 (US); Matsumoto, Hiroatsu, Zama-shi Kanagawa-ken (JP); Iwata, Shigemi, Yokohama-shi Kanagawa-ken (JP); Suzuki, Fujio, Galveston Texas 77554 (US); Utsunomiya, Tokuichiro, Yamato-shi Kanagawa-ken (JP); Takeda, Midori, Tokyo (JP)
(74) Representative: Dealtry, Brian

(57) **Abstract**

The object of the present invention is to provide the use of glycyrrhizin and its derivatives for induction of RANTES. The present invention discloses an RANTES induction method and pharmaceutical composition for the same comprising administration of glycyrrhizin and its derivatives in an amount effective for treating or preventing decreases in infection resistance to opportunistic infections occurring in burn patients, AIDS patients, cancer patients, encephalitis patients, individuals having suffered serious injuries or undergone major surgery, or individuals subject to stress.

## Description

### Cross-References to the Applications

This application claims the priority benefit of U.S. Provisional Application Ser. No. 60/422,339 filed October 29, 2002.

### BACKGROUND OF THE INVENTION

### Field of the Invention

The present invention relates to the use of glycyrrhizin and its derivatives for RANTES induction and the production of a RANTES inducer, a RANTES induction method, and a pharmaceutical composition for the same.

### Prior Art

Cytokines are proteins produced by lymphocytes and other cells, and act on cells having receptors to them to promote cell growth, differentiation and expression of function. In addition, cytokines also include a group of proteins referred to as chemokines that promote chemotaxis, namely the migration of cells. These chemokines have a common structure in which they have four cysteine residues, and are classified into subfamilies such as CXC, CC and so forth according to the motif formed by two cysteine residues on the N terminal side.

Chemokines belonging to the CXC subfamily have a sequence in which the first two cysteine residues on the N terminal side surround one amino acid, and induce chemotaxis in vitro particularly in neutrophils. On the other hand, chemokines belonging to the CC subfamily have an amino acid sequence in which the first two cysteine residues on the N terminal side are arranged in a row directly without having an amino acid between them, and are known to induce chemotaxis in vitro in monocytes, macrophages, T cells, NK cells, eosinophils and so forth.

RANTES is a type of chemokine belonging to the CC subfamily, is primarily involved in the migration of type 1 T cells and the activation of cytotoxic T cells, and play an important role in the infection resistance and antitumor resistance of individuals. Thus, if it were possible to induce the action of RANTES, it is believed that therapeutic effects would be obtained against diseases such as infections and tumors in which the type I T cell reaction plays a primary role in their defense.

However, there has yet to be reported an effective means of inducing RANTES. Therefore, the object of the present invention is to provide a RANTES induction method and a pharmaceutical composition for inducing RANTES.

### SUMMARY OF THE INVENTION

As a result of earnest studies to achieve the above object, the inventors of the present invention focused on glycyrrhizin (glycyrrhizinic acid) and its derivatives.

Glycyrrhizin (or glycyrrhizinic acid) is a compound composed of glycyrrhetic acid and two molecules of glucuronic acid, and has been known since long ago to have anti-inflammatory action. In addition, it is also known to have gastric juice secretion inhibitory action, digestive organ ulcer healing action, action that enhances antiallergic activity, immunosuppressive action, liver function enhancing action, detoxifying action and action that enhances resistance to viruses. In particular, it is a compound that widely used in the clinical setting as a liver disease agent and allergy agent.

Glycyrrhizin has been reported in recent years to have action that inhibits the intracellular growth of HIV, and have an effect that allows survival for 10 years or more without the onset of AIDS when glycyrrhizin is administered at 150 to 225 mg (6 to 9 tablets) per day to patients who are asymptomatic carriers (AC).

The inventors of the present invention found that glycyrrhizin and its derivatives have action that induces RANTES, and that they are effective in protection from septicemia, MRSA and other infections, thereby leading to completion of the present invention.

Namely, the present invention provides the use of glycyrrhizin and its derivatives for induction of RANTES.

The glycyrrhizin and its derivatives used in the present invention are represented with the following general formula (I): 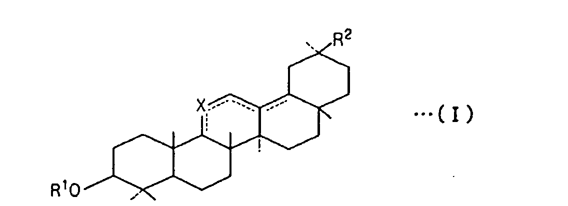 [wherein,

R¹ represents a hydrogen atom or a group of the following formula (II) or (III): 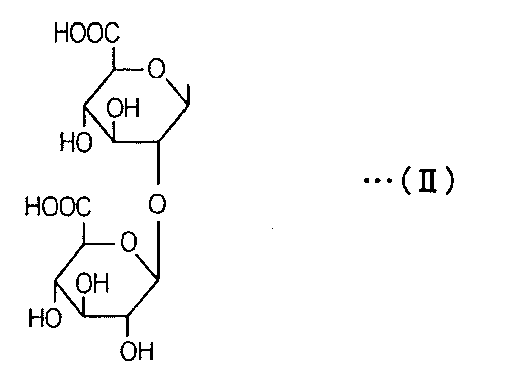 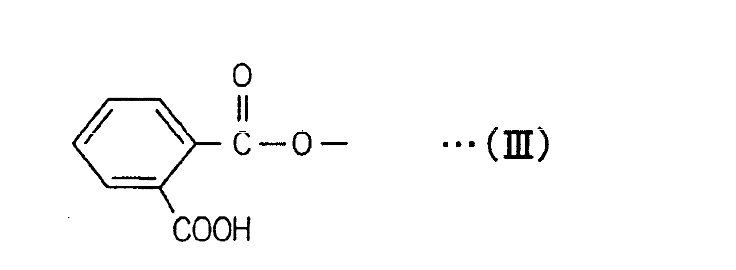 {wherein, the groups of formula (II) and formula (III) may also be their pharmaceutically acceptable salts);

R² represents COOH or a group of the following formula (IV): 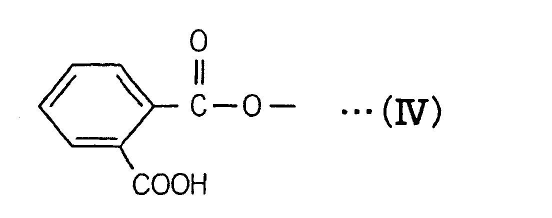 or their pharmaceutically acceptable salts;
X represents C=O or CH; and,
dotted lines suitably represent a double bond].

In the above compounds, the pharmaceutically acceptable salts in formulas (II), (III) and (IV) are sodium salts, potassium salts, calcium salts, magnesium salts, aluminum salts, ammonium salts or various other organic amine salts, but preferably are sodium salts, potassium salts, ammonium salts or combinations thereof.

The following compounds are included in the above general formula (I):
* sodium olean-3β-hydroxy-11-oxo-12-ene-30-ate;
* disodium olean-9(11),12-diene-3β,30-diol-3β,30-O-dihemiphthalate;
* disodium olean-11,13(18)-diene-3β,30-diol-3β,30-O-dihemiphthalate;
* disodium olean-3β-hydroxy-11,13(18)-diene-30-ate-3β-O-hemiphthalate;
* disodium olean-3β-hydroxy-11-oxo-12-ene-30-ate-3β-O-hemiphthalate; and
* monoammonium 20β-carboxy-11-oxo-30-norolean-12-en-3β-yl-2-O-β-D-glucopyranuronosyl-β-D-glucopyranosidouronate.

The present invention provides the use of the above compounds in the production of a RANTES inducer.

The RANTES induction method of the present invention is characterized by the administration of the above compounds in an amount effective for RANTES induction.

The pharmaceutical composition of the present invention is characterized by containing the above compounds along with an arbitrary pharmaceutically acceptable carrier in an amount effective for treating or preventing decreases in infection resistance to opportunistic infections occurring in burn patients, AIDS patients, cancer patients, encephalitis patients, individuals having suffered serious injuries or undergone major surgery, or individuals subject to stress.

### Detailed Description of the Preferred Embodiments

The glycyrrhizin and its derivatives used for RANTES induction in the present invention can be acquired from, for example, Minophagen Pharmaceutical. Alternatively, glycyrrhizin derivatives are described in, for example, Chem. Pharm. Bull. 34, 897 (1986) and Jpn. J. Pharmacol. , 71, 281 (1996).

Disodium olean-3β-hydroxy-11-oxo-12-ene-30-ate-3β-O-hemiphthalate can be synthesized in the manner described below.

### Synthesis Method of Disodium Olean-3β-Hydroxy-11-Oxo-12-Ene-30-Ate-3β-O-Hemiphthalate

300 ml of methanol and 30 ml of 1 N aqueous sodium hydroxide solution were added to 10 g of olean-11,13(18)-diene-3-hydroxy-30-ate-3-O-hemiphthalic acid, and stirred to dissolve. Aqueous sodium hydroxide solution was added during stirring so that the pH of the reaction solution stayed within the range of 10.0-10.4. After preparing the reaction solution, the solution was filtered using a membrane filter. Following filtration, the reaction solution was concentrated to about half of its original volume, 200 ml of acetone were added, and the white crystals that precipitated were recovered and dried. 9.3 g of the above compound, which is one of the compounds used in the present invention, were obtained. The melting point was 283-287°C, and the mass analysis value (m/z) was 601 (M-1).

### Synthesis Method of Olean-11,13(18)-Diene-3-Hydroxy-30-Ate-3-O-Hemiphthalic Acid

A mixture of 30 g of olean-11,13(18)-diene-3-hydroxy-30 acid, 60 g of phthalic anhydride and 2 g of 4-dimethylaminopyridine were added to 300 ml of chloroform, and refluxed for 24 hours at 80°C. Following completion of the reaction, the solvent was distilled off and 240 ml of ethanol were added to the residue. After dissolving by heating at 90°C, 240 ml of water were added followed by heating and stirring for an additional 15 minutes. After cooling, the precipitated white crystals were recovered, and 200 ml of 50% ethanol were added to these crystals after which heating and stirring were continued for 30 minutes. After cooling, the white crystals that did not dissolve were recovered by filtration, and then washed with 50% aqueous ethanol and dried to obtain 37.6 g of olean-11,13(18)-diene-3-hydroxy-30-ate-3-O-hemiphthalic acid.

### Synthesis Method of Olean-11,13(18)-Diene-3-Hydroxy-30 Acid

47.1 g of 18α-glycyrrhetic acid were dissolved in 500 ml of tetrahydrofuran (THF) and then mixed with 500 ml of 1 N aqueous sodium hydroxide solution and 500 ml of THF at 80°C, after which a solution containing 75.6 g of sodium borohydroxide was dropped in to this mixture and allowed to react for 24 hours at the same temperature. Following completion of the reaction, the reaction solution was returned to room temperature followed by the addition of 600 ml of acetone and stirring, and neutralization of the solution using 2 N hydrochloric acid. The THF in the reaction solution was distilled off, and the white crystals that precipitated (mixture of 11α-hydroxy- glycyrrhetic acid and 11β-hydroxy-glycyrrhetic acid) were recovered by filtration. After dissolving these crystals in 1000 ml of THF and drying, the solvent was distilled off and 400 ml of chloroform were added to the residue followed by recovery of the insoluble crystals by filtration and drying to obtain 40 g of a mixture of 11α-hydroxy-glycyrrhetic acid and 11β-hydroxy-glycyrrhetic acid. This mixture was dissolved in 4000 ml of THF followed by the addition of 1600 ml of 10% hydrochloric acid and stirring for 3 hours at room temperature. The white crystals that precipitated were recovered by filtration, washed with water and dried to obtain 35.8 g of olean-11,13(18)-diene-3-hydroxy-30 acid.

In addition, the glycyrrhizin and its derivatives used in the present invention can also be obtained from resources that exist in nature such as licorice having glycyrrhizin as one of its components or licorice powder, licorice extract or crude licorice extract obtained from licorice.

Although explained in detail in the examples to follow, glycyrrhizin and its derivatives have been determined to have the ability to induce RANTES by inducing RANTES production by acting on peripheral mononuclear cells, T cells, monocytes and so forth. Thus, the use of glycyrrhizin and its derivatives is a useful means for inducing RANTES, and glycyrrhizin and its derivatives can be used as the active ingredient of RANTES inducers.

The RANTES induction method of the present invention utilizes the ability of glycyrrhizin and its derivatives to induce RANTES, and involves the administration of an amount of glycyrrhizin and its derivatives that is effective for inducing RANTES. Here, an amount effective for inducing RANTES refers to the amount of glycyrrhizin and its derivatives that produces an adequate amount of RANTES for inducing migration of type 1 T cells and activation of cytotoxic T cells and so forth while also preventing problems (adverse side effects) and economic loss caused by excessive administration of glycyrrhizin and its derivatives.

In this manner, RANTES can be efficiently induced by suitably determining the amount of glycyrrhizin and its derivatives effective for induction of RANTES and administering that amount.

In the RANTES induction method of the present invention, glycyrrhizin and its derivatives are preferably used in the form of a pharmaceutical composition of the invention of the present application, namely a form that contains glycyrrhizin and its derivatives in an amount effective for treatment and prevention of the intended target diseases or target states in the present application (or control of the mechanisms of action that cause said diseases or states such as promotion of migration of type 1 T cells) and an arbitrary pharmaceutically acceptable carrier.

As has been explained above, according to the RANTES induction method of the present invention, since RANTES can be efficiently induced and produced, the migration of type 1 T cells and the function of effector cells in type 1 T cell reactions, such as cytotoxic T cells, macrophages and natural killer cells, are promoted, and the resistance of individuals to bacterial infections can be enhanced.

The following provides an explanation of a pharmaceutical composition of the present invention.

A pharmaceutical composition of the present invention contains an effective amount of glycyrrhizin and its derivatives for inducing RANTES, and glycyrrhizin and its derivatives may be contained alone, or mixed with an arbitrary pharmaceutically acceptable carrier, in said pharmaceutical composition. In addition, a pharmaceutical composition of the present invention can be prepared in various forms in accordance with ordinary methods, examples of said forms include tablets, injections, capsules, sprays, troches and powder. Furthermore, a "pharmaceutically acceptable carrier" refers to that which does not substantially inhibit the function of the active ingredient in said composition, and more specifically, includes solid diluents or fillers, sterile aqueous solvents and various nontoxic organic solvents.

Although a RANTES inducer or pharmaceutical composition obtained according to the present invention can be administered orally, rectally, locally, percutaneously, intravenously or intramuscularly, the administration route is suitably selected by the clinician corresponding to the weight and health status of the patient to be treated or prevented as well as the status of the disease to be treated. The dose can be administered, for example, in a single dose or divided among several doses at 0.1-100 mg per 1 kg of body weight per day.

According to a pharmaceutical composition of the present invention, since RANTES can be efficiently induced, it is able to induce migration of type I T cells and activation of effector cells of type I T cell reactions, and can be expected to allow the obtaining of therapeutic effects against diseases such as infections and tumors in which type I T cell reactions and their defense play a major role.

It is particularly preferable for the treatment or prevention of decreased infection resistance to opportunistic infections occurring in burn patients, AIDS patients, cancer patients, encephalitis patients, individuals having suffered serious injuries or undergone major surgery, or individuals subject to stress.

### Examples

### <RANTES Induction Ability of Glycyrrhizin and its Derivatives>

### Example 1

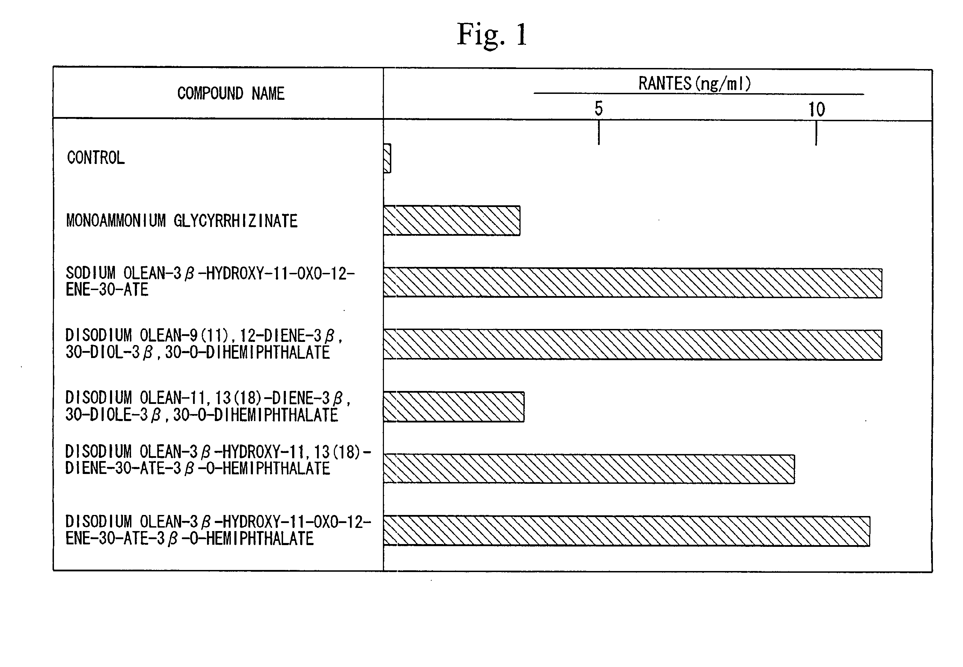

Peripheral blood was collected from healthy individuals and peripheral mononuclear cells were isolated with Ficoll-Hypaque. RPMI1640 medium containing 10% FCS and antibiotics (penicillin and streptomycin) was used for culturing. After stimulating 1 x 10⁶ cells/ml of peripheral mononuclear cells with 100 µg/ml of sodium olean-3β-hydroxy-11-oxo-12-ene-30-ate, disodium olean-9(11),12-diene-3β,30-diol-3β,30-O-dihemiphthalate, disodium olean-11,13 (18)-diene-3β,30-diol-3β,30-O-dihemiphthalate, disodium olean-3β-hydroxy-11,13(18)-diene-30-ate-3β-O-hemiphthalate, disodium olean-3β-hydroxy-11-oxo-12-ene-30-ate-3β-O-hemiphthalate, or monoammonium glycyrrhizinate for 24 hours, the amount of RANTES in the culture supernatant was measured with a Human RANTES/CCL5 Quantikine Immunoassay Kit (R&D Systems). Furthermore, the control in the drawing indicates the amount of RANTES in culture supernatant when peripheral mononuclear cells were treated with medium only. Those results are shown in Fig. 1.

As is clear from Fig. 1, in contrast to hardly any RANTES being detected with the control, in the culture supernatant stimulated by monoammonium glycyrrhizinate, 3 ng/ml of RANTES were induced, a nearly equal amount of RANTES was induced by disodium olean-11,13(18)-diene-3β,30-diol-3β,30-O-dihemiphthalate.

In addition, since a greater amount of RANTES is induced in culture supernatant stimulated by compounds other than those indicated above, these compounds were determined to have more potent ability to induce RANTES in peripheral mononuclear cells.

### Example 2

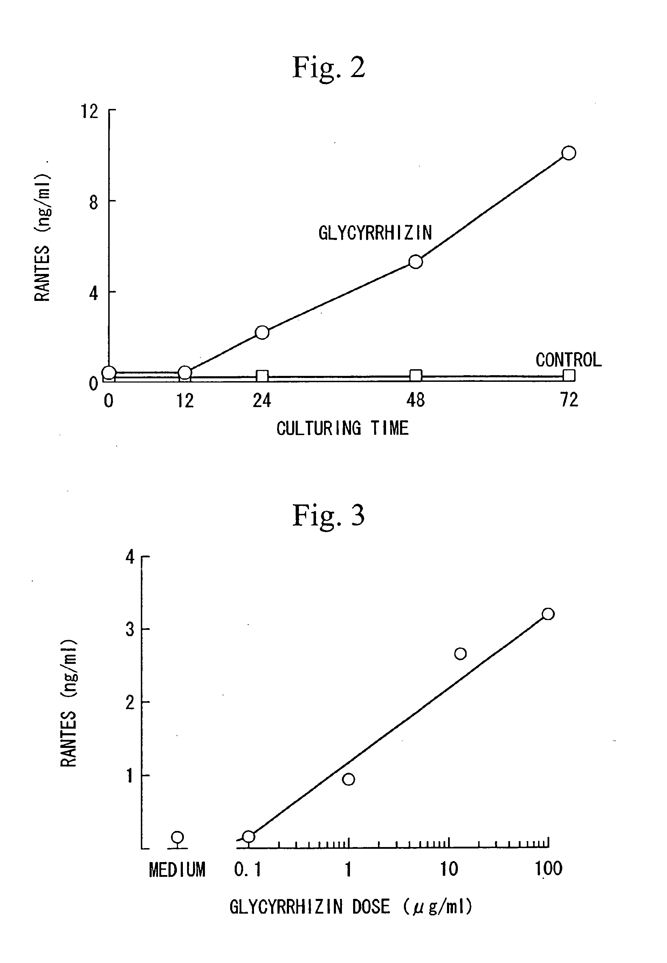

Peripheral blood was collected from healthy subjects and peripheral mononuclear cells were isolated with Ficoll-Hypaque. 1 x 10⁶ cells/ml of peripheral mononuclear cells were stimulated with 100 µg/ml of monoammonium glycyrrhizinate (monoammonium glycyrrhizinate will hereinafter be referred to as glycyrrhizin) . Taking the time at which glycyrrhizin was added to be 0 hours, the amount of RANTES present in the culture supernatant was measured with the Human RANTES/CCL5 Quantikine Immunoassay Kit (R&D Systems) 12, 24, 48 and 72 hours later. Those results are shown in Fig. 2. Furthermore, the control in the drawing indicates the amount of RANTES in culture supernatant of peripheral mononuclear cells not treated with glycyrrhizin.

As is clear from Fig. 2, the production of RANTES by mononuclear cells stimulated by glycyrrhizin increased with the passage of culturing time.

### Example 3

Peripheral blood was collected from healthy subjects and peripheral mononuclear cells were isolated with Ficoll-Hypaque. 1 x 10⁶ cells/ml of peripheral mononuclear cells were stimulated for 24 hours with 0.1-100 µg/ml of glycyrrhizin. The amount of RANTES present in the culture supernatant was measured with the Human RANTES/CCL5 Quantikine Immunoassay Kit (R&D Systems). Those results are shown in Fig. 3.

Glycyrrhizin induced RANTES in a dose dependent manner within the range of 1-100 µg/ml. The above results were able to be confirmed not only in humans, but also in experiments using mononuclear cells originated from mouse spleen.

### Example 4

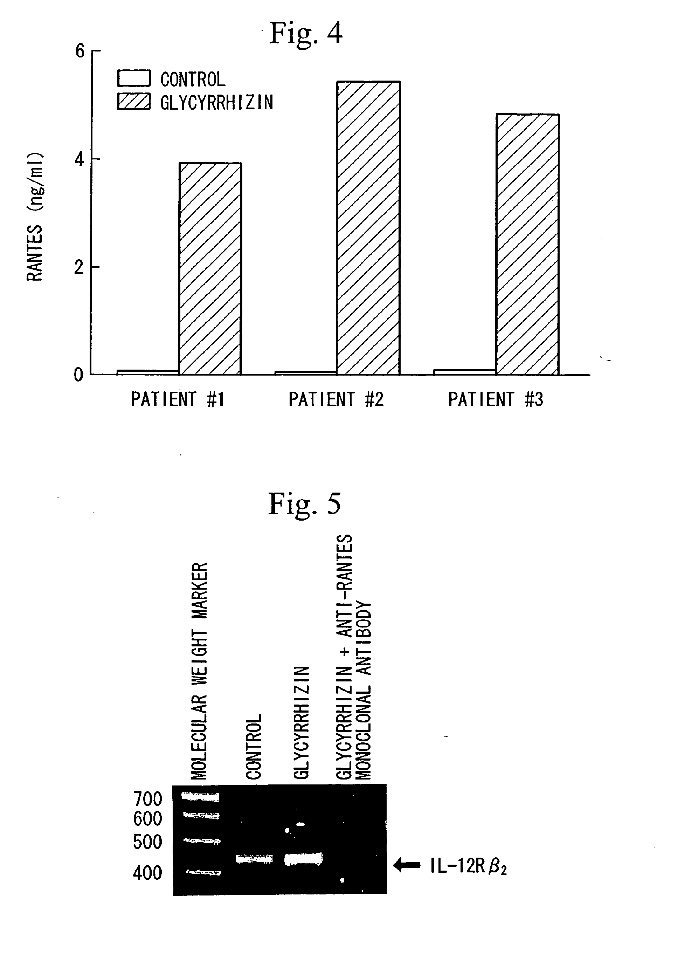

Peripheral blood was collected from 3 AIDS patients (CD4 count: 215-418 cells/µl, viral load: 80,000-370,000 copies/ml) and peripheral mononuclear cells were isolated with Ficoll-Hypaque. 1 x 10⁶ cells/ml of peripheral mononuclear cells were stimulated for 24 hours with 100 µg/ml of glycyrrhizin. The amount of RANTES present in the culture supernatant was measured with the Human RANTES/CCL5 Quantikine Immunoassay Kit (R&D Systems). Furthermore, the control in the drawing indicates the amount of RANTES in culture supernatant of peripheral mononuclear cells not treated with glycyrrhizin. Those results are shown in Fig. 4.

Similar to peripheral mononuclear cells originated from healthy individuals, RANTES was induced when peripheral mononuclear cells from AIDS patients were stimulated with glycyrrhizin.

### <Th1 Cell Induction Action>

### Example 5

Peripheral blood was collected from healthy subjects and peripheral mononuclear cells were isolated with Ficoll-Hypaque. 1 x 10⁶ cells/ml of peripheral mononuclear cells were cultured for 7 days in medium containing IL-2 (10 U/ml) and anti-IFN-γ monoclonal antibody (10 µg/ml) in the presence of 100 µg/ml of glycyrrhizin, or glycyrrhizin and anti-RANTES monoclonal antibody (10 µg/ml). After culturing, RNA was extracted from the cells, and the expression of IL-12Rβ2 mRNA, a marker for Th1 cells, was measured by RT-PCR. Furthermore, since glycyrrhizin is known to induce production of IFN-γ, in order to demonstrate that IFN-γ is not involved, the experiment was carried out in the presence of anti-IFN-γ monoclonal antibody. In other words, IFN-γ is not involved in the reaction used in this experiment.

Furthermore, the control in the drawing indicates the degree of expression of IL-12Rβ2 mRNA in mononuclear cells when cultured without the addition of glycyrrhizin. Those results are shown in Fig. 5.

Although cells stimulated with glycyrrhizin exhibited an increase in the expression of IL-12Rβ2 mRNA, since cells cultured with anti-RANTES monoclonal antibody no longer demonstrated its expression, the Th1 induction action of glycyrrhizin was demonstrated to be mediated by induction of RANTES.

### <Infection Defensive Effects>

### Example 6

As mentioned above, since glycyrrhizin is known to induce production of IFN-γ, an experiment was conducted using IFN-γ knockout mice to demonstrate that IFN-γ is not involved.

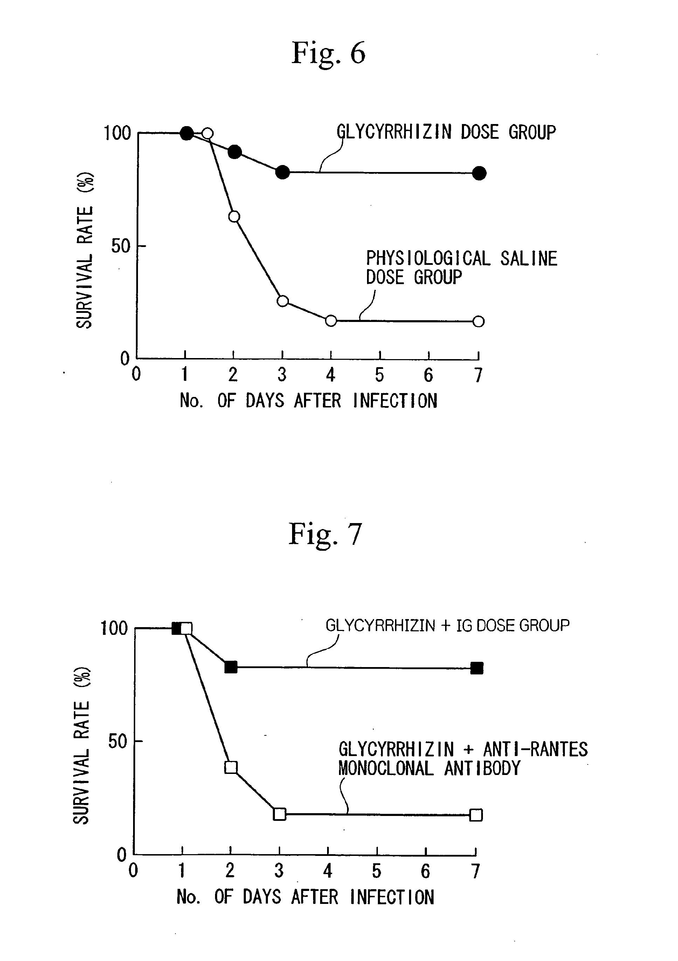

Glycyrrhizin was administered intraperitoneally to IFN-γ knockout mice at 20 mg/kg, and septicemia was induced by cecal ligation and puncture (CLP) 2 hours later. Furthermore, septicemia was also induced in the same manner 2 hours after administration of physiological saline to a control group. The survival rates (%) of the IFN-γ knockout mice 7 days following septicemic infection are shown in Fig. 6.

### Example 7

Glycyrrhizin was administered intraperitoneally to IFN-γ knockout mice at 20 mg/kg, and septicemia was induced by CLP 2 hours later. 10 µg/ml of anti-RANTES monoclonal antibody were administered subcutaneously 2 hours before, 12 hours after and 24 hours after administration of glycyrrhizin, while an equal amount of Ig was administered subcutaneously to a control group. The survival rates (%) of the IFN-γ knockout mice for 7 days after septicemic infection are shown in Fig. 7.

As is clear from the results shown in Fig. 6, in contrast to 80% of the IFN-γ knockout mice having died by 4 days after septicemic infection, 80% of all of the IFN-γ knockout mice administered glycyrrhizin avoided death and survived. However, according to the results of Fig. 7, since the mortality rate increased to the same level as the control group when anti-RANTES monoclonal antibody was administered with glycyrrhizin, the effect of glycyrrhizin against septicemia was indicated as appearing mediated by the induction of RANTES.

### Example 8

Spleen mononuclear cells originated from IFN-γ knockout mice (5 x 10⁶ cells/ml) were inoculated into SCIDbgMN mice (SCID-beige mice deficient in macrophage and neutrophil functions). A 20 mg/kg dose of glycyrrhizin was administered intraperitoneally immediately after inoculation. 2 hours later, the animals were infected through the tail vein with methicillin-resistant Staphylococcus aureus (MRSA) at a level of 5 LD₅₀. Furthermore, a control group was infected with an equal amount of MRSA following administration of physiological saline. The survival rates (%) of the SCIDbgMN mice for 10 days after MRSA infection are shown in Table 8.

### Example 9

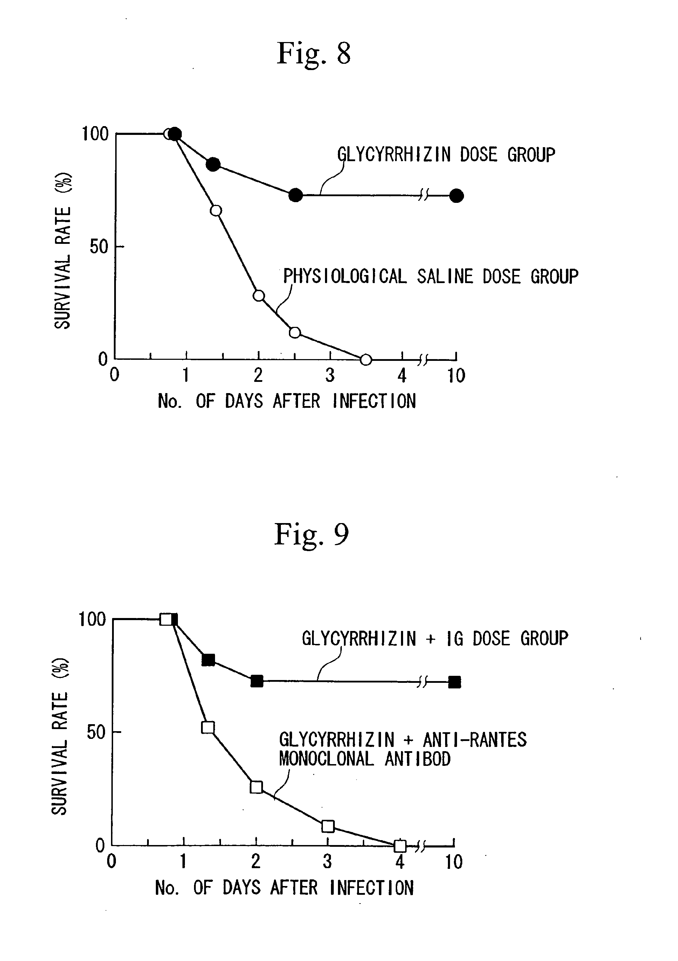

Spleen mononuclear cells originated from IFN-γ knockout mice (5 x 10⁶ cells/ml) were inoculated into SCIDbgMN mice. A 20 mg/kg dose of glycyrrhizin was administered intraperitoneally immediately after inoculation. 2 hours later, the animals were infected through the tail vein with methicillin-resistant Staphylococcus aureus (MRSA) at a level of 5 LD₅₀. 10 µg/ml of anti-RANTES monoclonal antibody was administered subcutaneously 2 hours before, 12 hours after and 24 hours after administration of glycyrrhizin, while an equal amount of Ig was administered subcutaneously to a control group. The survival rates (%) of the SCIDbgMN mice 10 days after MRSA infection are shown in Fig. 9.

As is clear from Fig. 8, in contrast to 100% of the physiological saline dose group having died by 3.5 days after MRSA infection, 75% of all animals in the glycyrrhizin dose group avoided death and survived. However, according to the results of Fig. 9, since the mortality rate increased to the same level as the control group when anti-RANTES monoclonal antibody was administered with glycyrrhizin, the defensive effect of glycyrrhizin against MRSA infection was indicated as appearing mediated by induction of RANTES.

The group of compounds synthesized in the present invention are shown below.

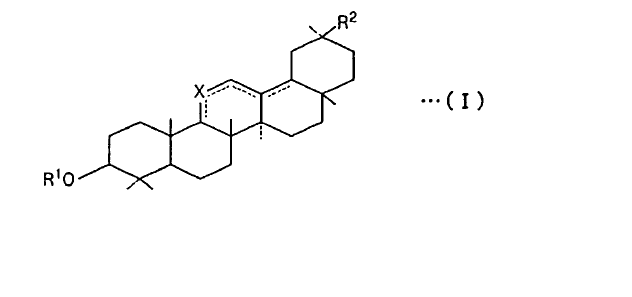

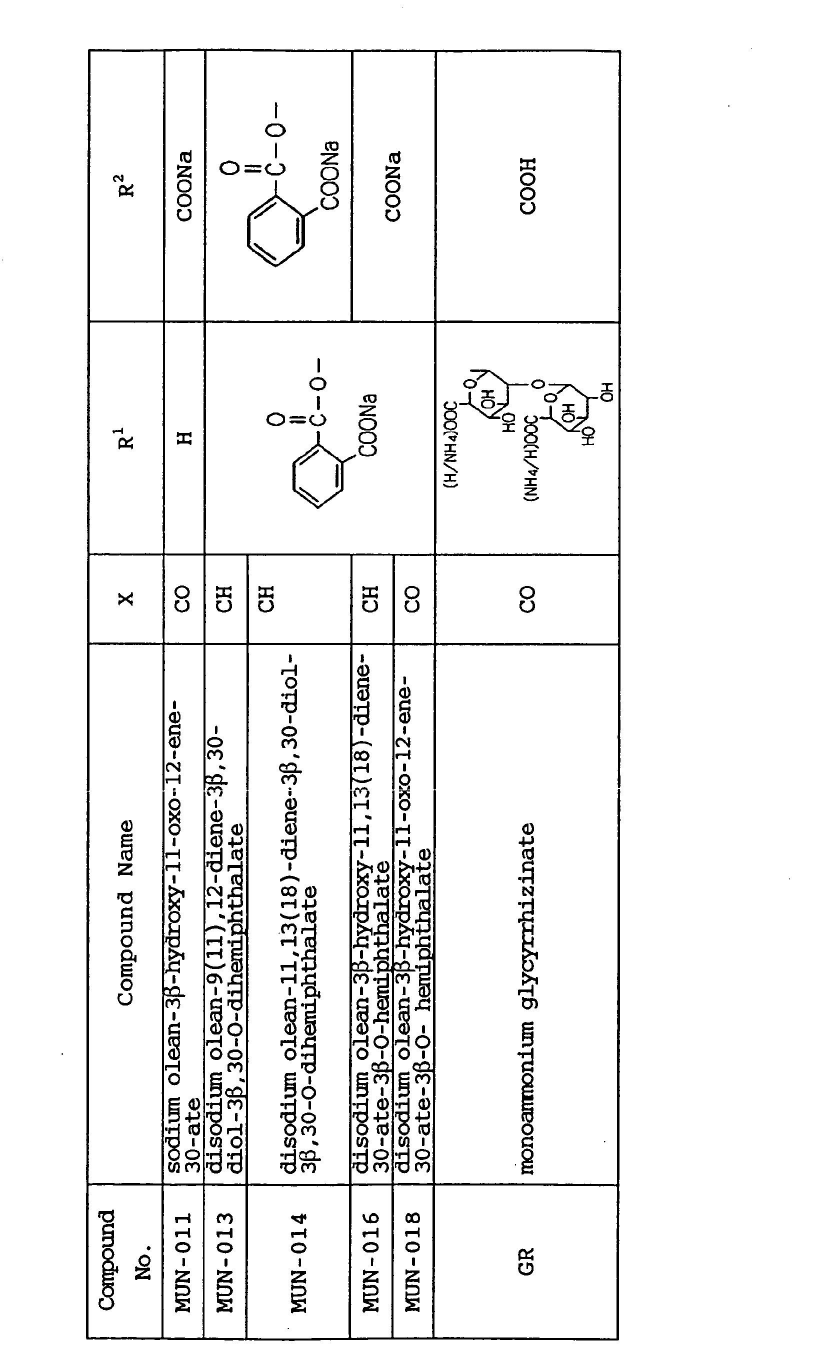

According to the present invention, RANTES can be induced by using glycyrrhizin and its derivatives.

According to the RANTES induction method of the present invention, RANTES can be induced efficiently.

Since the pharmaceutical composition of the present invention is able to induce RANTES efficiently, it can be preferably used for the treatment of infections and cancer by enhancing the migration of type 1 T cells, the activation of effecter cells in type 1 T cell reactions, and local type 1 T cell reactions of infections and tumors.

### BRIEF DESCRIPTION OF THE DRAWINGS

Fig. 1 shows the amounts of RANTES produced following stimulation of peripheral mononuclear cells with glycyrrhizin and its derivatives for 24 hours.
Fig. 2 shows the amounts of RANTES produced over time following stimulation of peripheral mononuclear cells with glycyrrhizin.
Fig. 3 shows the correlation between the amount of glycyrrhizin that acts on peripheral mononuclear cells and the amount of RANTES produced.
Fig. 4 shows the amounts of RANTES produced following stimulation of peripheral mononuclear cells originating in AIDS patients with glycyrrhizin for 24 hours.
Fig. 5 shows the action of induction of Th1 cells by glycyrrhizin (degree of expression of IL-12Rβ2 mRNA).
Fig. 6 shows the infection defensive effects of glycyrrhizin in a septicemia model.
Fig. 7 shows the effects of RANTES on the infection defensive effects of glycyrrhizin in a septicemic infection model.
Fig. 8 shows the infection defensive effects of glycyrrhizin against MRSA infection.
Fig. 9 shows the effects of RANTES on the infection defensive effects of glycyrrhizin against MRSA infection.

## Claims

1. The use of a compound represented with the following general formula (I) for RANTES induction: 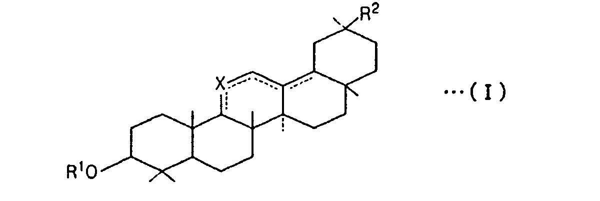 [wherein,
R¹ represents a hydrogen atom or a group of the following formula (II) or (III): 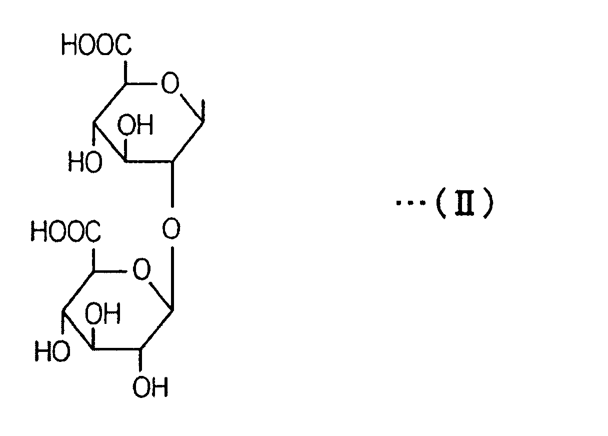 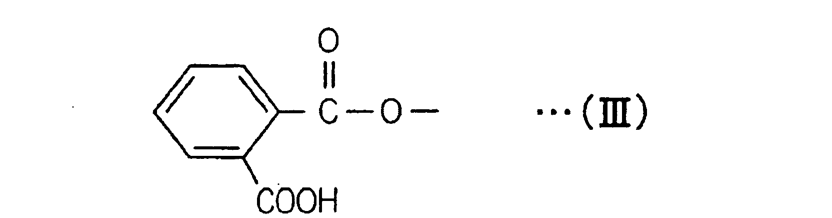 {wherein, the groups of formula (II) and formula (III) may also be their pharmaceutically acceptable salts);
R² represents COOH or a group of the following formula (IV): 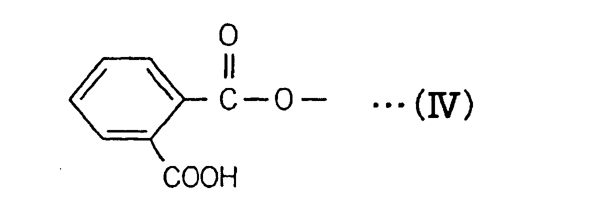 or their pharmaceutically acceptable salts;
X represents C=O or CH; and,
dotted lines suitably represent a double bond].

2. The use according to claim 1 wherein, the pharmaceutically acceptable salts in the above formulas (II), (III) and (IV) are sodium salts, potassium salts, ammonium salts or combinations thereof.

3. The use of a compound according to claim 1 wherein, a compound of the above general formula (I) is one of either:
sodium olean-3β-hydroxy-11-oxo-12-ene-30-ate;
disodium olean-9(11),12-diene-3β,30-diol-3β,30-O-dihemiphthalate;
disodium olean-11,13(18)-diene-3β,30-diol-3β,30-O-dihemiphthalate;
disodium olean-3β-hydroxy-11,13(18)-diene-30-ate-3β-O-hemiphthalate;
disodium olean-3β-hydroxy-11-oxo-12-ene-30-ate-3β-O-hemiphthalate; or
monoammonium 20β-carboxy-11-oxo-30-norolean-12-en-3β-yl-2-O-β-D-glucopyranuronosyl-β-D-glucopyranosidouronate.

4. The use of a compound according to any of claims 1 through 3 in the production of a RANTES inducer.

5. A RANTES induction method comprising: administration of a compound according to any of claims 1 through 3 in an amount effective for RANTES induction.

6. A pharmaceutical composition comprising: containing a compound according to claim 1 with an arbitrary pharmaceutically acceptable carrier in an amount effective for treating or preventing decreases in infection resistance to opportunistic infections occurring in burn patients, AIDS patients, cancer patients, encephalitis patients, individuals having suffered serious injuries or undergone major surgery, or individuals subject to stress.
